(19) Europäisches Patentamt / European Patent Office / Office européen des brevets

(11) **EP 1 512 729 B1**

(12) **EUROPEAN PATENT SPECIFICATION**

(45) Date of publication and mention
of the grant of the patent:
**10.01.2007 Bulletin 2007/02**

(21) Application number: **03723320.2**

(22) Date of filing: **12.05.2003**

(51) Int Cl.:
*C09D 11/00* (2006.01)     *C01B 33/12* (2006.01)
*C01B 13/14* (2006.01)     *C03B 37/005* (2006.01)
*C01F 7/02* (2006.01)     *C03C 14/00* (2006.01)
*C08K 3/22* (2006.01)     *C08L 101/00* (2006.01)
*A61K 8/00* (2006.01)

(86) International application number:
**PCT/JP2003/005868**

(87) International publication number:
**WO 2003/099944 (04.12.2003 Gazette 2003/49)**

(54) **SCALY PARTICLES AND COSMETIC COMPRISING THE SAME, COATING COMPOSITION, RESIN COMPOSITION AND INK COMPOSITION**

SCHUPPENFÖRMIGE TEILCHEN UND SIE ENTHALTENDES KOSMETIKUM, BESCHICHTUNGSZUSAMMENSETZUNG, HARZZUSAMMENSETZUNG UND TINTENZUSAMMENSETZUNG

PARTICULES ECAILLEUSES ET PRODUIT COSMETIQUE COMPRENANT CES PARTICULES, COMPOSITION DE REVETEMENT, COMPOSITION DE RESINE ET COMPOSITION D'ENCRE

(84) Designated Contracting States:
**DE FR GB IT**

(30) Priority: **24.05.2002 JP 2002149987**

(43) Date of publication of application:
**09.03.2005 Bulletin 2005/10**

(73) Proprietor: **Nippon Sheet Glass Co.,Ltd.
Tokyo 105-8552 (JP)**

(72) Inventor: **YOKOI, Koji
c/o Nippon Sheet Glass Company Ltd.
Tokyo 105-8552 (JP)**

(74) Representative: **Albrecht, Thomas
Kraus & Weisert,
Thomas-Wimmer-Ring 15
80539 München (DE)**

(56) References cited:
**EP-A2- 0 268 938     JP-A- 1 009 803
JP-A- 1 143 821     JP-A- 6 116 119
JP-A- 8 026 931     JP-A- 8 040 831
JP-A- 63 307 142**

• **PATENT ABSTRACTS OF JAPAN vol. 1996, no. 04, 30 April 1996 (1996-04-30) & JP 07 315859 A (NIPPON SHEET GLASS CO LTD), 5 December 1995 (1995-12-05)**

## Description

FIELD OF THE INVENTION

The present invention relates to flaky particles to be blended as a filler into a cosmetic, a coating material, a resin, a film, or an ink. The present invention also relates to a cosmetic, a coating material, a film, a resin composition, a resin form, or an ink each containing the flaky particles.

BACKGROUND OF THE INVENTION

Filler which scatters transmitted light or reflected light to make the ground to be hardly seen (make a shade of the ground) while keeping high transmission of visible light so as to keep the brightness has been used in a field of cosmetics, kneaded resins, films, inks, and paints. The filler has an effect of providing great dispersion of visible light so as to exhibit dullness-free translucent white color. As such filler, for example for cosmetics, coating materials, resins and the like, spherical fine particles such as barium sulfate and silica of which particle size is controlled are disclosed in JP H08-225316A and JP H08-59436A. However, since the spherical fine particles are easily agglomerated, a cosmetic, a coating matieral, a resin or the like containing the spherical fine particles may give gritty feeling (feeling of larger particles). Therefore, there are problems of bad touch and poor usability. There is also a problem of reducing the function of scattering transmitted light or reflected light when the spherical fine particles are agglomerated. Accordingly, there are some cases that some dullness appears while white is kept because of scattered light.

For the purpose of solving the problems due to the agglomeration and the purpose of effectively exhibiting the scattering, spherical inorganic fine particles coated with microparticles have been developed (JP H08-217637A, JP H11-1411A etc.). Microparticles are attached to inorganic fine particles for the purpose of preventing the mircoparticles from being agglomerated. However, since the inorganic fine particles are spherical, the inorganic fine particles are easily agglomerated in themselves, thus leading to poor scattering effect as a result. When the fine particles are blended in a cosmetic, there are problems of bad touch and poor usability similarly to the above. There another problem that the microparticles attached to the surfaces of the inorganic fine particles easily come off due to mechanical friction.

JP H10-87433A illustrates a filler comprising plate-like fine particles coated with microparticles. Since the plate-like fine particles are difficultly agglomerated in themselves, a cosmetic using this filler is expected to provide smooth touch without giving gritty feeling. However, the filler remains the problem that the microparticles easily come off because the microparticles are attached to the plate-like fine particles.

JP H05-39436A describes a technique of scattering transmitted light using plate-like fine particles having butterfly configuration. However, it is quite difficult to manufacture the plate-like fine particles having butterfly configuration and the tolerance of manufacturing conditions is very small so that there is a problem that the management of manufacturing conditions must be clearly defined. When oil solution for cosmetic, resin, and ink having refractive index close to that of the plate-like fine particles is used, there is a problem that the scattering of transmitted light is reduced or substantially vanished.

JP S63-126818A describes a laminar substance containing metallic compound microparticles of 5-500 nm in diameter dispersed therein, for example, a laminar silica containing titanium oxide particles of 30 nm in diameter. While the laminar substance can screen ultraviolet light, it can not effectively scatter visible light.

DISCLOSURE OF THE INVENTION

The first object of the present invention is to provide flaky particles suitable for a filler having high durability and high weatherability which absorbs reduced amount of visible light and effectively scatters visible light. The second object of the present invention is to provide a cosmetic capable of giving good usability such as spreading well, exhibiting bright and clear appearance for long period, and making spots and pores of skin to be hardly seen, by using the flaky particles to be contained as a filler of the cosmetic. The third object of the present invention is to provide a coating material which spreads well on a substrate, makes the ground to be hardly seen, and exhibits dullness-free translucent white color. The fourth object of the present invention is to provide a resin compact and an ink having high scattering effect or having translucent white color.

Flaky particles of the present invention comprise mother particles of metallic oxide of low refractive index and microparticles of metallic oxide of high refractive index having a mean particle size of 160-450 nm dispersed inside the mother particles in an amount of 5-50% by weight, wherein the flaky particles have a light diffusion degree of 80 or more.

The flaky particles can be used as a filler for cosmetic or resin composition. Since the profiles of the particles are flaky, the particles are hardly agglomerated. Therefore, the flaky particles have no problem of poor usability such as gritty feeling for example when the flaky particles are blended into cosmetic.

The flaky particles contain microparticles having particle size of 160-450 nm, preferably 200-400 nm, dispersed therein. The particle size of the microparticles is about 1/2 of visible light wavelength (360-830 nm) . Microparticle has greatest impact on light passing near the microparticle when the particle size of the microparticle is half of the wavelength of the target light. The flaky particles containing microparticles having such particle size dispersed therein can effectively reflect or refract visible light passing through the near of the microparticles. Therefore, the flaky particles can haze (conceal) the ground under the flaky particle while keeping low visible light absorptivity and high visible light reflectivity. The flaky particles can exhibit dullness-free translucent white color. In case that the finish form is thin film form such as a cosmetic, a coating material, a resin composition for film, and an ink composition, the flaky particles therein are directed within the thin film to be parallel to the surface of the thin film, thereby increasing the aforementioned effect.

If the cosmetic, the coating material, the resin composition for film, or the ink composition contains metallic oxide particles having high refractive index of 160-450 nm in particle size which are not dispersed inside the flaky particles, the metallic oxide particles are agglomerated to form secondary particles of larger particle size. On the surface of the metallic oxide particles having thus formed secondary particles, reflection and refraction of visible light is hardly repeated, thus making the concealing effect poor. The cosmetic contains microparticles which are not dispersed inside the flaky particles has poor spreading property and thus poor usability because the microparticles are agglomerated. The coating material containing microparticles which are not dispersed inside the flaky particles provides poor spread when blending.

The flaky particles preferably has a mean particle size of 5-500 $\mu$m, a mean thickness of 0.1-5 $\mu$m, and a mean aspect ratio of 5-300, more preferably has a mean particle size of 8-300 $\mu$m, a mean thickness of 0.2-2.5 $\mu$m, and a mean aspect ratio of 8-200, and further preferably has a mean particle size of 8-50 $\mu$m, a mean thickness of 0.5-2.0 $\mu$m, and a mean aspect ratio of 8-50. The mean particle size of the flaky particles can be measured by a laser diffraction/ scattering technique particle size distribution analyzer, for example, MICROTRAC 2 (available from NIKKISO CO., LTD). The mean thickness can be obtained as a simple average of 50 flaky particles measured by an electron microscope. The mean aspect ratio can be calculated by dividing a value of the mean particle size with a value of the mean thickness. When the mean particle size is less than 5 $\mu$m, the flaky particles are easily agglomerated so that the scattering of visible light hardly occurs. On the other hand, when the mean particle size exceeds 500 $\mu$m, the flaky particles are easily broken when blended as a filler. Further, when the flaky particles having mean particle size exceeding 500 $\mu$m are blended into a cosmetic, the cosmetic has poor usability such as giving gritty feeling. Flaky particles having mean thickness less than 0.1 $\mu$m have problems that the production was difficult and the flaky particles are easily broken. On the other hand, when flaky particles having mean thickness exceeding 5 $\mu$m are blended into a coating material, irregularities are formed in the surface of coating layer made of the coating material so that the appearance is poor. When the flaky particles having mean thickness exceeding 5 $\mu$m are blended into a cosmetic, the cosmetic has poor usability such as giving gritty feeling. Flaky particles having mean aspect ratio less than 5 start to show the feature as spherical particles, that is, the flaky particles are easily agglomerated. On the other hand, flaky particles having mean aspect ratio exceeding 300 are easily broken when blended as a filler.

As the microparticles of metallic oxide having high refractive index and having particle size of 160-450 nm, microparticles having refractive index higher than the refractive index of mother particles of the flaky particles are used. The refractive index of the microparticles is preferably higher than the refractive index of the mother particles of the flaky particles by 0.5 or more, especially preferably by 1.0 or more. Since the scattering of visible light occurs at boundary of microparticles, when the difference between the refractive index of the microparticles and the refractive index of mother particles is 0.5 or more, the scattering of visible light is increased so that the flaky particles have a degree of light diffusion of 80 or more. The degree of light diffusion means a value for total luminous of visible light. Increase in degree of light diffusion means increase in light scattering not only of total transmitted light but also of total reflected light (decrease in direct reflected light).

The material of the microparticles is preferably at least one selected from a group consisting of zinc oxide (ZnO, 1.9-2.1 in refractive index), titanium dioxide (TiO$_2$, rutile, 2.76 in refractive index; anatase, 2.52 in refractive index), zirconium dioxide (ZrO$_2$, 2.1-2.2 in refractive index), cerium oxide (CeO$_2$, 2.2 in refractive index), aluminum sesquioxide (Al$_2$O$_3$, 1.6-1.8 in refractive index), antimony sesquioxide (Sb$_2$O$_3$, 2.0-2.3 in refractive index), tin oxide (SnO$_2$, 2.0 in refractive index), and iron sesquioxide (Fe$_2$O$_3$, 2.9-3.2 in refractive index). Particles of metallic oxide having high visible light absorptivity such as black color Fe$_3$O$_4$, FeO and low-order titanium oxide are not preferable even though the particles have high refractive index of 160-450 nm in particle size. Among the aforementioned particles, titanium dioxide, zirconium dioxide, cerium oxide, and iron sesquioxide have ultraviolet lay absorbing capability. In case of using these particles, the particles provide effect as ultraviolet-lay blocking agent and further provide additional effect. Examples as particles made of one of these materials include, as titanium dioxide particles. "TIPAQUE CR-60" of 210 nm in mean particle size, available from ISHIHARA SANGYO CO., LTD., "TIPAQUE CR-50" of 250 nm in mean particle size, available from ISHIHARA SANGYO CO., LTD., "TIPAQUE CR-58" of 280 nm in mean particle size, available from ISHIHARA SANGYO CO., LTD., and "KR-460" of 300 nm, available from TITAN KOGYO CO., LTD.; as zinc oxide (ZnO) particles, "microparticle zinc oxide SF-15" of 180 nm in mean particle size, available from SAKAI CHEMICAL INDUSTRY CO., LTD.; and as iron sesquioxide (Fe$_2$O$_3$) particles, "FRO-20" of 200 nm in mean particle size, available from SAKAI CHEMICAL INDUSTRY CO.,LTD. These microparticles can be manufactured by a liquid-phase hydrolysis method of conducting condensation polymerization under suitable conditions after the hydrolysis of metal alkoxide, a thermal decomposition method of metal alkoxide, a method of hydrolyzing metal chloride in flame, or a homogeneous precipitation method of hydrolyzing into solution of metal salt and adding a reagent of producing precipitating agent to cause reaction.

The mother particles preferably contain at least one selected from a group of silicon dioxide (1.46 in refractive index) and aluminum sesquioxide (1.6-1.8 in refractive index) in an amount of 50% by weight or more.

Preferable combinations of mother particles of flaky particles and microparticles are follows indicated as mother particles (refractive index)/microparticles (refractive index):

Silicon dioxide (1.46 in refractive index)/titanium dioxide (2.72 in refractive index);
Aluminum oxide (1.76 in refractive index)/iron sesquioxide (3.01 in refractive index);
Silicon dioxide (1.46 in refractive index)/zinc oxide (2.1 in refractive index);
Silicon dioxide (1.46 in refractive index)/ zirconium oxide (2.1 in refractive index);
Silicon dioxide (1.46 in refractive index) / cerium oxide (2.2 in refractive index);
Silicon dioxide (1.46 in refractive index) / iron sesquioxide (3.01 in refractive index).

The content of microparticles in flaky particles is 5-50% by weight, preferably 8-30% by weight. When the content is less than 5% by weight, the scattering of visible light is insufficient. On the other hand, when the content exceeds 50% by weight, the flaky particles must be brittle and have poor mechanical strength. The configuration of the microparticles is not particularly limited and may be amorphous, spherical, cylindrical, or fusiform-shaped.

The flaky particles preferably have a total light reflectitivy of 40% or more.

The flaky particles can be produced by a process of milling melt glass mixed with metal oxide particles having high refractive index into a film shape or a so-called sol-gel process, but the production method is not limited to these. Among these, the sol-gel process is especially suitable. For example, a method described in JP H01-9803A may be employed. That is, the method comprises preparing a stock solution in which microparticles are dispersed into a metallic compound solution or a metallic oxide sol (material of mother particles) prepared from metal alkoxide or metallic organic acid salt, applying the stock solution to a smooth surface to form a coating film, drying the coating film, making the coating film into laminar state by treatment such as reaction, and releasing the laminar metallic compound from the smooth surface. Instead of the metal alkoxide or the organic acid salt, a commercially available metallic oxide colloide solution may be employed as the stock sol of mother particles. The released laminar metallic compound is burned at a temperature of from 400°C to 1,200°C and, if necessary, is pulverized and classified so as to obtain flaky particles having a given mean particle size. When the temperature for the burning is relatively low, for example from 400°C to 800°C, porous flaky particles are obtained. When the temperature for the burning is relatively high, for example more than 800°C, dense, not porous, flaky particles are obtained. The porous flaky particles have relatively low strength due to the pores. However, since the refractive index (air=1) of the pores is significantly smaller than that of the mother particles, the improved scattering of visible light can be expected because of the boundaries of pores. The porous flaky particles are thus suitably used in applications not being subjected to much stress but requiring higher scattering intensity. The dense flaky particles are suitably used in applications requiring relatively higher strength and applications in which there will be a problem when the pores absorb peripheral substance.

Since the flaky particles contain microparticles dispersed inside thereof, the microparticles would never be exfoliated from the flaky particles even when the flaky particles are blended as a filler in any application.

As the flaky particles are blended into a cosmetic, the cosmetic thus prepared are excellent in clearness and soft focus effect (effect of blurring profiles of the ground by diffused reflection of light) and is pleasant to the touch. The proper content of the flaky particles in the cosmetic ranges from 1 to 70% by weight. When the content is less than 1% by weight, the soft focus effect should be insufficient. On the other hand, when the content exceeds 70% by weight, the soft focus property and the gloss should be too intensive, giving affected finish. The more preferable content ranges from 3 to 50% by weight.

The flaky particles may be suitably subjected to hydrophobization treatment. As the method of hydrophobization treatment, a treatment with a silicone compound such as methyl hydrogen polysiloxane, high-viscosity silicone oil, or silicone resin, a treatment with a surfactant such as anion activator or cation activator, a treatment with high polymer compound such as nylon, polymethyl methacrylate, polyethylene, fluorocarbon resin, or polyamino acid, or a treatment with a compound containing perfluoro group, lecithin, collagen, metal soap, lipophilic wax, polyalcohol partial ester, or complete ester, and a complex treatment as combination thereof. The method may be any method which can be applied for hydrophobization treatment and is thus not limited to the aforementioned treatments.

Besides the flaky particles, other components usually used in cosmetics may be suitably blended into the cosmetic, if necessary. Examples of the other components include inorganic powders, organic powders, pigments, coloring matters, oily components, organic solvents, resins, and plasticizers. Specific examples of inorganic powders are talc, kaolin, sericite, white mica, golden mica, red mica, black mica, lithia mica, vermiculite, magnesium carbonate,

**EP 1 512 729 B1**

calcium carbonate, diatomaceous earth, magnesium silicate, calcium silicate, aluminum silicate, barium silicate, barium sulfate, strontium silicate, metal tungstate salt, silica, hydroxyapatite, zeolite, boron nitride, and ceramic powder.

Examples of organic powders are nylon powder, polyethylene powders, polystyrene powders, benzoguanamine powder, polytetrafluoroethylene powder, distyrene benzene polymer powders, epoxy powder, and acrylic powder.

Examples of the pigments are inorganic white pigments such as microcrystalline cellulose, titanium dioxide and zinc oxide, inorganic red pigments such as iron oxide (colcothar) and iron titanate, inorganic brown pigments such as γ-iron oxide, inorganic yellow pigments such as yellow iron oxide and ocher, inorganic black pigments such as black iron oxide and carbon black, inorganic purple pigments such as manganese violet and cobalt violet, inorganic green pigments such as chromium oxide, chromium hydroxide, and cobalt titanate, inorganic blue pigments such as Prussian blue and ultramarine blue, pearl pigments such as titanium oxide-coated mica, titanium oxide-coated bismuth oxychloride, bismuth oxychloride, titanium oxide-coated talc, scales foil and titanium oxide-coated colored mica, and metallic powder pigments such as aluminum powders and copper powders.

Examples of the coloring matters are organic pigments such as red No. 201, red No. 202, red No. 204, red No. 205, red No. 220, red No. 226, red No. 228, red No. 405, orange No. 203, orange No. 204, yellow No. 205, yellow No. 401 and blue No. 404, organic pigments of zirconium, barium and aluminum lake such as red No. 3, red No. 104, red No. 106, red No. 227, red No. 230, red No. 401, red No. 505, orange No. 205, yellow No. 4, yellow No. 5, yellow No. 202, yellow No. 203, green No. 3 and blue No. 1, and natural colorants such as chlorophyll and β-carotene.

Examples as the oily components are squalene, liquid paraffin, Vaseline, microcrystalline wax, ozokerite, ceresine, myristic acid, palmitic acid, stearic acid, oleic acid, isostearic acid, cetyl alcohol, hexadecyl alcohol, oleyl alcohol, cetyl 2-ethylhexanoate, 2-ethylhexyl palmitate, 2-octyldodecyl myristate, neopentyl glycol di-2-ethylhexanoate, glycerol tri-2-ethylhexanoate, 2-octyldodecyl oleate, isopropyl myristate, glycerol triisostearate, glycerol tripalmitatem, olive oil, avocado oil, yellow beeswax, myristyl myristate, hydro carbones such as mink oil and lanolin, silicone oil, higher fatty acid, esters such as fat and oil, fatty alcohol, and wax.

Examples of the other components are an organic solvent such as acetone, toluene, butyl acetate or acetic ester, a resin such as alkyd resin or urea resin, a plasticizer such as camphor or acetyl tributyl citrate, ultraviolet absorber, antioxidant, preservative, surfactant, humectant, perfume, water, alcohol, and thickener.

The cosmetics may take on various forms including powder, cake-like, pencil-like, stick-like, ointment-like, liquid-phase, emulsion or cream-like forms. More specifically, the cosmetics include facial skin care cosmetics such as skin lotion, skin milk and cream, and makeups such as foundation, lip stick, eye shadow, brush-on, eye liner, nail enamel, and mascara.

The flaky particles can be used as a filler for a coating material, a kneaded resin, a film, or an ink similarly to conventional fillers. In case of using as a filler for a coating material, the resultant coating material can spread well on a coating substrate, make the ground to be hardly seen, and exhibit dullness-free translucent white color. In case of using as a filler for a resin form, a resin film, or an ink, the resultant product can exhibit higher scattering effect of visible light or exhibit dullness-free translucent white color. The content of the flaky particles in the coating material composition, the resin composition, or the ink composition preferably ranges from 1 to 70% by weight. When the content is less than 1% by weight, the visible light scattering effect should be insufficient so that it is impossible to exhibit the ground concealing effect and translucent white color. On the other hand, when the content exceeds 70% by weight, the gloss should be too intensive. The more preferable content ranges from 3 to 50% by weight.

BRIEF DESCRIPTION OF THE DRAWINGS

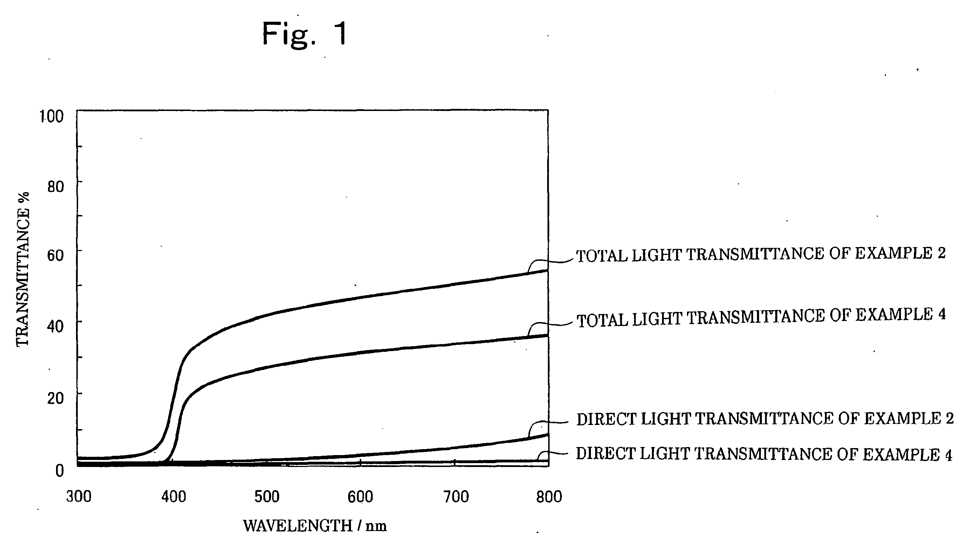

Fig. 1 is a graph showing spectral characteristics including the respective total light transmittances and the respective direct light transmittances at a wavelength of 300-800 nm of flaky particles of examples of the present invention.

DESCRIPTION OF THE PREFERRED EMBODIMENTS

[Examples]

Hereinafter, the present invention will be described in detail with reference to the following examples. It should be noted that the present invention is not limited to the following examples.

As for flaky particles prepared in the respective examples and comparative examples, the scattering of visible light was evaluated by the following means.

5

[Evaluation method of scattering of visible light]

Flaky particles are added into vinyl chloride resin coating material (Vinyroze clear GA00011, available from DAI NIPPON TORYO CO., LTD., the solvent is a mixture of toluene, xylene, and butyl acetate, the solid matter is 50% by weight, the refractive index of resin is 1.54) in such a manner that the content of the flaky particles in the resin is 10% by weight. After mixing and agitating them well, the mixture is applied to a substrate and then dried. The dried mixture is peeled off from the substrate so as to form a film of 100 $\mu$m in thickness. As for each thus formed film, the direct light transmittance (Dt), as the parallel light transmittance, and the total light transmittance (Tt) at a wavelength of 550 nm that is the most sensitive wavelength to human's eyes are measured using a spectrophotometer (U3100, available from Hitachi, Ltd.) and a standard light A as a light source according to JIS K-7105-1981 5.5.2, respectively. In conducting the measurement, the direct light transmittance and the total light transmittance of a vinyl chloride resin film without addition of flaky particles are previously measured and the data of the films containing flaky particles are calibrated in such a manner that the direct light transmittance and the total light transmittance of the former are set to be 100%. Value obtained by subtracting the direct light transmittance (Dt) from the total light transmittance (Tt) is scattered light transmittance (St). Based on the values of the direct light transmittance and the total light transmittance, light diffusion degree H is obtained by the following equation. The larger light diffusion degree H shows the larger scattering of visible light.

```
Light diffusion degree H = ((total light transmittance Tt -
direct light transmittance Dt) / total light transmittance
Tt) × 100
= (Scattered light transmittance St / total light
transmittance Tt) × 100
```

As for each of the films, the total light reflectance at a wavelength of 550nm is also measured by a spectrophotometer (U3100, available from Hitachi, Ltd.) and a standard light A as a light source according to JIS K-7105-1981 5.5.3. In conducting the measurement, the total light reflectance of a vinyl chloride resin film without addition of flaky particles are measured and the data of the films containing flaky particles are calibrated in such a manner that the total light reflectance of the former is set to be 0%.

(Examples 1-6)

670g of colloidal silica containing silica, i.e. silicon dioxide, of about 30% by weight (SILICADOL-30A, available from Nippon Chemical Industrial Co., Ltd., the particle diameter is 20nm, the dispersion medium is water), 500g of ethanol, and 500g of water were mixed. Microparticles of titania, i.e. titanium dioxide (without dispersion medium) of various particle sizes shown in Table 1 were added into the mixture in a predetermined amount and were dispersed uniformly using a beads mill so as to prepare silica sol solution containing titania microparticles. A stainless steel plate of a square 10 cm on a side was dipped into the solution and, according to the dipping method, the aforementioned solution was applied to the stainless steel plate in such a manner as to obtain a coating layer of 1.0 $\mu$m in dried state. After that, the stainless steel plate was entered into a dry kiln of 120°C for 5 minutes to dry the coating layer. Then, the coating layer was peeled off by a scraper so as to obtain flakes. The obtained flakes were sintered at a temperature of 1000°C for 2 hours, thereby obtaining dense flaky particles made from silica in major proportions and containing dispersed titania microparticles. The flaky particles were classified by a known apparatus and adjusted to have a mean particle size of 15 $\mu$m, a mean thickness of 1.0 $\mu$m, and a mean aspect ratio of 15.

As for the flaky particles, the visible light transmittance (total light transmittance) and the scattering of visible light were measured and evaluated according to the aforementioned manner. The results including the mean particle size ($\mu$m), the mean thickness ($\mu$m), and the mean aspect ratio of the flaky particles, the mean particle size (nm) and the content (% by weight) of titania particles dispersed in the flaky particles, the titania raw material, the visible light transmittance (%), and the light diffusion degree are shown in Table 1. As for the flaky particles of any of the examples, the visible light transmittance (%) ranges from 30 to 45% and the light diffusion degree exceeds 90. It is found from the results that these flaky particles very effectively scatter light. As for Examples 2 and 4, spectral characteristics of the total light transmittances and the direct light transmittances at wavelength from 300 to 800 nm are shown in Fig. 1. It is found from this graph that ultraviolet rays are effectively blocked because there is steep decline in total light transmittance under wavelength of 400 nm.

Table 1

| Example | 1 | 2 | 3 | 4 | 5 | 6 |
|---|---|---|---|---|---|---|
| Mean particle size ($\mu$m) | 15 | 15 | 15 | 15 | 15 | 15 |
| Mean particle thickness($\mu$m) | 1 | 1 | 1 | 1 | 1 | 1 |
| Mean aspect ratio | 15 | 15 | 15 | 15 | 15 | 15 |
| Titania raw material | 1 | 2 | 2 | 2 | 3 | 4 |
| Particle size of titania (nm) | 210 | 250 | 250 | 250 | 280 | 300 |
| Content of titania | 20 | 10 | 20 | 30 | 20 | 20 |
| Total light transmittance (%) | 38 | 45 | 36 | 30 | 33 | 30 |
| Light diffusion degree H | 95 | 94 | 95 | 98 | 98 | 98 |
| Total light reflectance (%) | 62 | 55 | 64 | 70 | 67 | 70 |

Note) Titania raw material 1: TIPAQUE CR-60 available from ISHIHARA SANGYO CO., LTD.
2: TIPAQUE CR-50 available from ISHIHARA SANGYO CO., LTD.
3: TIPAQUE CR-58 available from ISHIHARA SANGYO CO., LTD.
4: KR-460 available from TITAN KOGYO KABUSHIKI CO

(Comparative Examples 1-3)

Flaky particles were made in the same manner as Example 1 except that titania microparticles of particle sizes shown in Table 2 were used instead of the titania raw material used in Example 1, respectively. The visible light transmittance (total light transmittance) and the scattering of visible light were measured. The results are shown in Table 2.

Table 2

| Comparative Example | 1 | 2 | 3 |
|---|---|---|---|
| Mean particle size ($\mu$m) | 15 | 15 | 15 |
| Mean particle thickness ($\mu$m) | 1 | 1 | 1 |
| Mean aspect ratio | 15 | 15 | 15 |
| Titania raw material | 5 | 5 | 6 |
| Particle size of titania (nm) | 110 | 110 | 150 |
| Content of titania (%) | 20 | 30 | 30 |
| Total light transmittance (%) | 77 | 70 | 66 |
| Light diffusion degree H | 65 | 69 | 75 |
| Total light reflectance (%) | 23 | 30 | 34 |

Note) Titania raw material 5: FA-80 available from FURUKAWA CO., LTD.
6: TIPAQUE A-100 available from ISHIHARA SANGYO CO., LTD.

It is found that when the mean particle size of microparticles is less than 160 nm even though the flaky particles contain dispersed titania microparticles, the measured light diffusion degree is 75 or less, that is, the scattering of visible light is significantly reduced.

(Example 7)

1,000g of colloidal alumina containing alumina, i.e. aluminum oxide, of about 20% by weight (ALUMINAZOL-520, available from Nissan Chemical Industries, Ltd.), 500g of ethanol, and 500g of water were mixed. 50g of microparticles of titania having a mean particle size of 250 nm (TIPAQUE CR-50 available from ISHIHARA SANGYO CO., LTD.) were added into the mixture and were dispersed uniformly using a beads mill so as to prepare alumina sol solution containing titania microparticles. A stainless steel plate of a square 10 cm on a side was dipped into the solution and, according to the dipping method, the aforementioned solution was applied to the stainless steel plate in such a manner as to obtain a coating layer of 1.0 $\mu$m in dried state. After that, the stainless steel plate was entered into a dry kiln of 120°C for 5 minutes to dry the coating layer. Then, the coating layer was peeled off by a scraper so as to obtain flakes. The obtained flakes were sintered at a temperature of 1000°C for 2 hours, thereby obtaining flaky particles made from alumina in major proportions and containing dispersed titania microparticles. The content of titania microparticles in the flaky particles was 20% by weight. The flaky particles were classified by a known apparatus and adjusted to have a mean particle size of 80 $\mu$m, a mean thickness of 1.0 $\mu$m, and a mean aspect ratio of 80.

As for the flaky particles, the visible light transmittance (total light transmittance), the light diffusion degree H and the total light reflectance were obtained according to the aforementioned method for evaluating the scattering of visible light. Since the light diffusion degree of the flaky particles was 90, it was found that the flaky particles quite effectively scatter light. The total light transmittance was 38% and the total light reflectance was 62%.

(Comparative example 4)

Flaky particles made from alumina in major proportions and containing dispersed titania microparticles were made in the same manner as Example 7 except that titania microparticles having a mean particle size of 110 nm (FA-8 available from FURUKAWA CO., LTD.) were used instead of the titania microparticles used in Example 7. The light diffusion degree H of the obtained flaky particles was 60. This value is very smaller than that of Example 7. The total light transmittance was 70% and the total light reflectance was 30%.

(Example 8)

A mixture of 877g of tetraethoxysilane (Silicon tetra-ethoxide, available from TAMA CHEMICALS CO., LTD), 110g of water, 8ml of 60% nitric acid, and 500g of ethanol was cured in a closed vessel at 50°C for 15 hours. After that, 10.5g of iron oxide ($Fe_2O_3$) microparticles of 200 nm in particle size (available from TODA KOGYO CORP.) were added into the mixture and were dispersed uniformly using a beads mill so as to prepare silica sol solution containing iron oxide microparticles. A stainless steel plate of a square 10 cm on a side was dipped into the solution and, according to the dipping method, the aforementioned solution was applied to the stainless steel plate in such a manner as to obtain a coating layer of 1.0 $\mu$m in dried state. After that, the stainless steel plate was entered into a dry kiln of 120°C for 5 minutes to dry the coating layer. Then, the coating layer was peeled off by a scraper so as to obtain flakes. The obtained flakes were sintered at a temperature of 800°C for 2 hours, thereby obtaining flaky particles made from silica in major proportions and containing dispersed iron oxide microparticles. The content of iron oxide microparticles in the flaky particles was 5% by weight. The flaky particles were classified by a known apparatus and adjusted to have a mean particle size of 80 $\mu$m, a mean thickness of 1.0 $\mu$m, and a mean aspect ratio of 80. As for the flaky particles, the visible light transmittance (total light transmittance) and the light diffusion degree H were obtained according to the aforementioned method for evaluating the scattering of visible light. Since the light diffusion degree of the flaky particles was 90, it was found that the flaky particles quite effectively scatter light. The total light transmittance was 44% and the total light reflectance was 50%.

(Examples 9-12)

Flaky particles were made in the same manner as Example 1 except that microparticles of zinc oxide (ZnO, point refractive index ranging from 1.9 to 2.1 in refractive index, the same is true for the following), zirconium dioxide ($ZrO_2$, 2.1-2.2 in refractive index), cerium oxide ($CeO_2$, 2.2 in refractive index), and tin oxide ($SnO_2$, 2.0 in refractive index) were used instead of the titania raw material used in Example 1. The visible light transmittance (total light transmittance) and the scattering of visible light were measured. The results are shown in Table 3.

Table 3

| Example | 9 | 10 | 11 | 12 |
|---|---|---|---|---|
| Mean particle size ($\mu$m) | 15 | 15 | 15 | 15 |
| Mean particle thickness ($\mu$m) | 1 | 1 | 1 | 1 |
| Mean aspect ratio | 15 | 15 | 15 | 15 |
| Raw material of microparticles | ZnO | $ZrO_2$ | $CeO_2$ | $SnO_2$ |
| Particle size of microparticles (nm) | 290 | 200 | 300 | 300 |
| Content of microparticles (%) | 20 | 10 | 20 | 30 |
| Total light transmittance (%) | 38 | 50 | 40 | 35 |
| Light diffusion degree H | 90 | 85 | 89 | 88 |
| Total light reflectance (%) | 62 | 49 | 57 | 63 |

Cosmetics were made using the flaky particles prepared in the above examples and comparative examples. For these cosmetics, sensory evaluation about the usability was conducted. Items of the sensory evaluation are three, that is, finish, translucency, and soft focus effect of blurring skin. The cosmetics were evaluated on 5-point scale of 1 to 5 about the respective items. The evaluation standards for the respective items are shown in Table 4.

Table 4

| | Evaluation Finish | Translucency | Soft focus effect of blurring skin |
|---|---|---|---|
| 1 | Very unnatural | None | None |
| 2 | Unnatural | little | little |
| 3 | Middling | Middling | neutral |
| 4 | Acceptably natural | Slightly translucent | Slightly effective |
| 5 | Natural | Very high | Very effective |

For the sensory evaluation of the cosmetics, 10 panelists were employed. The usability was evaluated based on the average in evaluation of the 10 panelists. For facilitating understanding of the evaluation results, the following marks are used in tables described below.

◎ ... not lower than 4.5 and not higher than 5.0
○ ... not lower than 3.5 and lower than 4.5
● ... not lower than 2.5 and lower than 3.5
Δ ... not lower than 1.5 and lower than 2.5
✕ ... not lower than 1.0 and lower than 1.5

(Example 13: powder foundation)

Powder foundation was prepared from the following components (% by weight):

| | | | |
|---|---|---|---|
| (1) | Titanium oxide | 7 |
| (2) | Talc | 20 |
| (3) | White mica | 3 |
| (4) | Flaky particles of Example 1 | 55 |
| (5) | Nylon powder | 2 |
| (6) | Red iron oxide | 0.5 |
| (7) | Yellow iron oxide | 1 |
| (8) | Black iron oxide | 0.1 |
| (9) | Silicone oil | 1 |

(continued)

| | | |
|---|---|---|
| (10) | 2-ethylhexyl palmitate | 9 |
| (11) | Sorbitan sesquiolate | 1 |
| (12) | Preservative | 0.3 |
| (13) | Perfume | 0.1 |

The components (1) - (8) were mixed by a Henschel mixer. To this mixture, the components (9)- (13) dissolved and mixed by heat were added and then pulverized by a pulverizer. The pulverized matter was molded into a middle plate of 5.3 mm in diameter at a pressure of 160 kg/cm$^2$, thereby obtaining a powder foundation.

(Comparative Example 5)

A powder foundation was prepared in the same manner as Example 13 except that the flaky particles of Comparative Example 1 were used instead of the component (4) among the components of Example 13.
The results of the sensory evaluation tests for Example 13 and Comparative Example 5 are both shown in Table 5.

Table 5

| | Finish | Translucency | Soft focus effect of blurring skin |
|---|---|---|---|
| Example 13 | ○ | ○ | ◎ |
| Comparative Example 5 | ○ | ○ | Δ |

It is found from Table 5 that the powder foundation according to the present invention has excellent soft focus effect of blurring skin.

(Example 14: Powder spray)

Powder spray was prepared from the following components (% by weight):

| | | |
|---|---|---|
| (1) | Aluminum chlorohydrate | 30.0 |
| (2) | Flaky particles of Example 2 | 20.0 |
| (3) | Siliconized talc | 15.0 |
| (4) | Triclosan | 0.1 |
| (5) | Isopropyl myristate | 21.9 |
| (6) | Dimethyl polysiloxane | 10.0 |
| (7) | Sorbitan fatty acid ester | 3.0 |
| (13) | Perfume | proper quantity |

A mixture of the components (1)-(8) was entered into an aerosol vessel. A valve was attached to the vessel and the vessel was filled with aerosol propellant.

(Comparative Example 6)

Powder spray was prepared in the same manner as Example 14 except that the flaky particles of Comparative Example 2 were used instead of the component (2) among the components of Example 14.
The results of the sensory evaluation tests for Example 14 and Comparative Example 6 are both shown in Table 6.

Table 6

| | Finish | Translucency | Soft focus effect of blurring skin |
|---|---|---|---|
| Example 14 | ○ | ○ | ◎ |
| Comparative Example 6 | ○ | ○ | Δ |

It is found from Table 6 that the powder spray according to the present invention has excellent soft focus effect of blurring skin.

(Example 15: Oily stick foundation)

Oily stick foundation was prepared from the following components (% by weight):

| | | |
|---|---|---|
| (1) | Flaky particles of Example 3 | 13.0 |
| (2) | Titania | 7.0 |
| (3) | Kaolin | 20.0 |
| (4) | Talc | 2.0 |
| (5) | Mica | 3.3 |
| (6) | Red iron oxide | 1.0 |
| (7) | Yellow iron oxide n | 3.0 |
| (8) | Black iron oxide | 0.2 |
| (9) | Hard paraffin | 3.0 |
| (10) | Microcrystalline wax | 7.0 |
| (11) | Vaseline | 15.0 |
| (12) | Dimethylpolysiloxane | 3.0 |
| (13) | Squalane | 5.0 |
| (14) | Isopropyl palmitate | 17.0 |
| (15) | Antioxidant | proper quantity |
| (16) | Perfume | proper quantity |

The components (9) - (15) were dissolved at a temperature of 85°C. To thus obtained solution, the components (1)-(8) were added and mixed by a dispersion mill. Then, the mixture was dispersed by a colloid mill. After that, the component (16) was added. After deaeration, the mixture was poured into a vessel at a temperature of 70°C and was cooled, thereby obtaining an oily stick foundation.

(Comparative Example 7)

An oily stick foundation was prepared in the same manner as Example 15 except that the flaky particles of Comparative Example 2 were used instead of the component (1) among the components of Example 15.
The results of the sensory evaluation tests for Example 15 and Comparative Example 7 are both shown in Table 7.

Table 7

| | Finish | Translucency | Soft focus effect of blurring skin |
|---|---|---|---|
| Example 15 | ○ | ○ | ◎ |
| Comparative Example 7 | ○ | ○ | Δ |

It is found from Table 7 that the oily stick foundation according to the present invention has excellent soft focus effect of blurring skin.

(Example 16: emulsion foundation)

An emulsion foundation was prepared from the following components (% by weight):

| | | |
|---|---|---|
| (1) | Stearic acid | 0.4 |
| (2) | Isostearic acid | 0.3 |
| (3) | Cetyl 2-ethylhexanoate | 4 |
| (4) | Liquid paraffin | 11 |
| (5) | Polyoxymethylene-10-Stearyl Ether | 2 |

(continued)

| (6) | Talc | 8 |
| (7) | Pigment | 4 |
| (8) | Cetyl alcohol | 0.3 |
| (9) | Preservative | 0.07 |
| (10) | Flaky particles of Example 3 | 10 |
| (11) | Triethanolamine | 0.42 |
| (12) | Propylene glycol | 5 |
| (13) | Preservative | 0.02 |
| (14) | Ion-exchange water | 54.19 |
| (15) | Perfume | 0.3 |

After the components (1)-(9) were dissolved with heat at 85˚C and mixed, the component (10) was added and uniformly dispersed. A mixture obtained by dissolving the components (11)-(14) with heat at 85˚C and mixing them was gradually added to the former mixture so as to emulsify. After agitation with keeping the temperature to be the same as the temperature during emulsification for 10 minutes, the mixture was cooled to 45˚C with being continuously agitated. After addition of the component (15), the mixture was cooled to 35 ˚C with being continuously agitated. Products were taken out and were packed into a vessel, thereby obtaining an emulsion foundation.

(Comparative Example 8)

An emulsion foundation was prepared in the same manner as Example 16 except that the flaky particles of Comparative Example 2 were used instead of the component (10) among the components of Example 16.
The results of the sensory evaluation tests for Example 16 and Comparative Example 8 are both shown in Table 8.

Table 8

| | Finish | Translucency | Soft focus effect of blurring skin |
|---|---|---|---|
| Example 16 | ○ | ○ | ◎ |
| Comparative Example 8 | ○ | ○ | Δ |

It is found from Table 8 that the emulsion foundation according to the present invention has excellent soft focus effect of blurring skin.

(Example 17: Blush-on)

A blush-on was prepared from the following components (% by weight):

| (1) | Kaolin | 19.0 |
| (2) | Flaky particles of Example 8 | 5.0 |
| (3) | colcothar | 0.3 |
| (4) | Red No. 202 | 0.5 |
| (5) | Ceresin | 15.0 |
| (6) | Vaseline | 20.0 |
| (7) | Liquid paraffin | 25.0 |
| (8) | Isopropyl myristate ester | 15.0 |
| (9) | Antioxidant | proper quantity |

The components (1)-(4) were added to a part of the component (7) and were treated by a roller so as to prepare a pigment compound. On the other hand, the component (4) was dissolved into a part of the component (10) so as to prepare a dye compound. The compounds (5)-(9) were dissolved with heat at 90˚C. To this mixture, the pigment compound was added and uniformly dispersed by a homo mixer. After the dispersion, the mixture was packed into a predetermined vessel, thereby obtaining an objective brush-on.

The obtained brush-on was excellent all in finish, translucency, and soft focus effect of blurring skin.

(Example 18: Lipstick)

A lip stick was prepared from the following components (% by weight):

| | | |
|---|---|---|
| (1) | Hydrocarbon wax | 20 |
| (2) | Candelilla Wax | 3 |
| (3) | Glyceryl isostearate | 40 |
| (4) | Liquid paraffin | 26.8 |
| (5) | Titanium dioxide | 4 |
| (6) | Flaky particles of Example 4 | 4 |
| (7) | Organic pigment | 4 |
| (8) | Perfume | 0.2 |

The above components (1)-(4) were dissolved with heat at 85˚C. After the components (5)-(7) were agitated and mixed into the mixture, the component (8) was further mixed and agitated. Thus obtained mixture was packed in a predetermined vessel, thus obtaining a lipstick.

The obtained lipstick was excellent all in finish, translucency, and soft focus effect of blurring skin.

(Example 19: Eye shadow)

An eye shadow was prepared from the following components (% by weight):

| | | |
|---|---|---|
| (1) | Talc | 21 |
| (2) | White mica | 20 |
| (3) | Flaky particles of Example 4 | 40 |
| (4) | Pigment | 12 |
| (5) | Squalene | 4 |
| (6) | Cetyl 2-ethylhexanoate | 1.9 |
| (7) | Sorbitan sesquiolate | 0.8 |
| (8) | Preservative | 0.1 |
| (9) | Perfume | 0.2 |

The above components (1)-(4) were mixed by a Henschel mixer. A mixture made by mixing the components (5)-(9) with heat was blow-mixed to the former mixture and then pulverized. The pulverized matter was discharged into a middle plate, thereby obtaining an eye shadow.

Then, coating materials were prepared by using flaky particles of the aforementioned examples and comparative examples.

(Example 20: White coating composition)

A coating composition was prepared from the following components.

First, the following composition (parts by weight) was dispersed by a paint shaker for 60 minutes to prepare a dispersed vehicle.

| | | |
|---|---|---|
| (1) | Alkyd resin varnish | 20.6 |
| (2) | Melamine resin varnish | 10.6 |
| (3) | Swasol | 15.6 |
| (4) | Flaky particles of Example 4 | 15.6 |

To this dispersed vehicle,

| | | |
|---|---|---|
| (5) | Alkyd resin varnish | 26.3 |

(continued)

(6)  Melamin resin varnish  11.3

(parts by weight) were added and agitated, thereby producing a white coating composition.

(Comparative Example 9)

A white coating composition was produced in the same manner as Example 20 except that powder of pigment-grade titanium oxide (CR-50 available from ISHIHARA SANGYO CO., LTD.) was used instead of the flaky particles as the component (4) in the coating composition of Example 20. The amount of the titanium oxide was set to be 30% (that is, 4.7 parts by weight) relative to the amount of the flaky particles of the component (4) of Example 20 so that the content of titanium oxide in the coating composition was set to be the same as that of Example 20 because the content of titanium oxide in the flaky particles of Example 4 was 30% by weight.

As for the white coating compositions of Example 20 and Comparative Example 9, the hiding power at a thickness of 30 $\mu$m was measured according to the hiding power - Black and white paper chart method of JIS K5600-4-1 and the color was judged by eyes. The results are shown in Table 9.

Table 9

|  | Hiding power | Color |
| --- | --- | --- |
| Example 20 | 92 | Dullness-free translucent |
| Comparative Example 9 | 89 | Slightly dull white |

It is found from Table 9 that the white coating composition according to the present invention has high hiding power and high effect of concealing the ground and has fairly dullness-free translucent white color.

(Example 21: Recoating composition)

A polished steel plate of a square 300 mm on a side was coated with an umber rust-preventive coating material (Helgon, available from NIPPON PAINT CO., LTD.). The coating material of Example 20 was further applied to the rust-preventive coating layer so as to have another coating layer of about 50 $\mu$m in thickness. An yellow coating material (Unipon 200, available from NIPPON PAINT CO., LTD.) was further applied to the coating layer so as to have another coating layer of 30 $\mu$m in thickness. As the outer surface of the coating layer was observed by eyes, it was estimated that the coating layer exhibited fairly dullness-free yellow and there was no effects of the umber color of the rust-preventive coating material as the base coat.

(Comparative Example 10)

A steel plate with multiple coating layers having a structure similar to Example 21 was produced in the same manner as Example 21 except that the coating material of Comparative Example 9 was employed instead of the coating material of Example 20. Similarly to Example 21, as the outer surface of the coating layer was observed by eyes, it was estimated that the coating layer exhibited slightly dull yellow and there was effect of the umber color of the rust-preventive coating material as the base coat.

(Example 22: Resin composition and Resin form)

98% by weight of methyl methacrylate copolymerization beads and 2% by weight of the flaky particles of Example 4 were agitated and mixed by a Henschel mixer so as to obtain a resin composition. By using this resin composition, an acrylic resin form of 0.5 mm in thickness was produced by an extruder. The light diffusion degree H of the resin compact was 90.

(Comparative Example 11)

An acrylic form compact of 0.5 mm in thickness was produced in the same manner as Example 22 except that the flaky particles of Comparative Example 2 were used in the same amount instead of the flaky particles used in Example 22. The light diffusion degree H of the resin compact was 58.

It is found from Example 22 and Comparative Example 11 that the resin form of the present invention exhibits well light diffusion property.

(Example 23: Ink composition)

A white ink was prepared by sufficiently mixing the following components (% by weight):

| | | |
|---|---|---|
| (1) | Flaky particles of Example 4 | 12 |
| (2) | Ketone resin | 19 |
| (3) | Ethanol | 59 |
| (4) | Propylene glycol monomethyl ether | 10 |

As characters were written on a black paper by using this ink composition, the written charactors exhibit fairly dullness-free white color and there were no effects of the black color of the base paper.

Industrial Applicability

Since the flaky particles of the present invention contains microparticles dispersed therein of which particle size corresponds to 1/2 of visible light wavelength, the flaky particles absorb reduced amount of visible light and effectively scatter visible light. Since the microparticles are dispersed inside of the flaky particles, the microparticles never be exfoliated from the flaky particles not to deteriorate the characteristics of the flaky particles when the flaky particles are used in various applications. When the flaky particles are used as a filler of a cosmetic, a coating material, a resin compact, or an ink, the flaky particles have no problem of reducing usability because the particles would never be agglomerated in the cosmetic, and the cosmetic can brightly burr the skin and exhibit dullness-free white color.

**Claims**

1. Flaky particles comprising:

    particles of metallic oxide of low refractive index; and
    microparticles of metallic oxide having a high refractive index and a mean particle size of 160-450 nm dispersed inside the particles of low refractive index in an amount of 5-50% by weight,

    wherein the flaky particles have a light diffusion degree of 80 or more and a total light transmittance at 550 nm of under 50%,
    wherein the high refractive index of the microparticles is higher than the low refractive index of the particles of low refractive index by 0.5 or more and
    wherein the light diffusion degree is defined by:

$$\text{Light diffusion degree } H = ((\text{total light transmittance } Tt - \text{direct light transmittance } Dt) / \text{total light transmittance } Tt) \times 100 = (\text{Scattered light transmittance } St / \text{total light transmittance } Tt) \times 100.$$

2. Flaky particles as claimed in claim 1, wherein the flaky particles have a mean particle size of 5-500 $\mu$m, a mean thickness of 0.1-5 $\mu$m, and a mean aspect ratio of 5-300.

3. Flaky particles as claimed in claim 1, wherein the flaky particles have a mean particle size of 8-300 $\mu$m, a mean thickness of 0.2-2.5 $\mu$m, and a mean aspect ratio of 8-200.

4. Flaky particles as claimed in claim 1, wherein the flaky particles have a mean particle size of 8-50 $\mu$m, a mean thickness of 0.5-2.0 $\mu$m, and a mean aspect ratio of 8-50.

5. Flaky particles as claimed in any one of claims 1 to 4, wherein the flaky particles have a total light reflectance of 40% or more.

6. Flaky particles as claimed in any one of claims 1 to 5, wherein the major component of the microparticles is at least one selected from the group consisting of zinc oxide (ZnO), titanium dioxide ($TiO_2$), zirconium oxide ($ZrO_2$), cerium oxide ($CeO_2$), aluminum oxide ($Al_2O_3$), antimony oxide ($Sb_2O_3$), and iron oxide ($Fe_2O_3$).

7. Flaky particles as claimed in any one of claims 1 to 6, wherein the major component of the particles of low refractive index containing the microparticles is at least one selected from the group consisting of silicon dioxide and aluminum sesquioxide.

8. Flaky particles as claimed in any one of claims 1 to 6, wherein the high refractive index of the microparticles is higher than the low refractive index of the particles of low refractive index by 1.0 or more.

9. Flaky particles as claimed in any one of claims 1 to 8, wherein the combination of the particles of low refractive index and the microparticles is at least one selected from the group consisting of the combinations, indicated as particles of low refractive index (refractive index)/microparticles (refractive index):

   Silicon dioxide (1.46 in refractive index)/titanium dioxide (2.72 in refractive index);
   Aluminum oxide (1.76 in refractive index)/iron sesquioxide (3.01 in refractive index);
   Silicon dioxide (1.46 in refractive index)/zinc oxide (2.1 in refractive index);
   Silicon dioxide (1.46 in refractive index)/zirconium oxide (2.1 in refractive index);
   Silicon dioxide (1.46 in refractive index)/cerium oxide (2.2 in refractive index);
   Silicon dioxide (1.46 in refractive index)/iron sesquioxide (3.01 in refractive index).

10. Flaky particles as claimed in any one of claims 1 to 9, wherein the content of the microparticles in flaky particles ranges from 8 to 30% by weight.

11. Flaky particles as claimed in any one of claims 1 to 10, wherein the mean particle size of the microparticles ranges from 200 to 400 nm.

12. Flaky particles as claimed in any one of claims 1 to 11, wherein the flaky particles are a film-shaped, milled mixture of melt glass and metal oxide particles having a high refractive index, or a sol-gel product.

13. A cosmetic containing flaky particles as claimed in any one of claims 1 to 12.

14. A cosmetic as claimed in claim 13, wherein the content of the flaky particles is 1-70% by weight.

15. A coating composition comprising flaky particles as claimed in any one of claims 1 to 12.

16. A coating composition as claimed in claim 15, wherein the content of the flaky particles is 1-70% by weight.

17. A coating layer comprising a hardened application of the coating composition as claimed in claim 15 or 16.

18. A resin composition containing flaky particles as claimed in any one of claims 1 to 12.

19. A resin composition as claimed in claim 18, wherein the content of the flaky particles is 1-70% by weight.

20. A resin form produced by molding the resin composition as claimed in claim 18 or 19.

21. An ink composition containing flaky particles as claimed in any one of claims 1 to 12.

22. An ink composition as claimed in claim 21, wherein the content of the flaky particles is 1-70% by weight.

23. Flaky particles as claimed in claim 1, wherein said total light transmittance is above 30%.

**Patentansprüche**

1. Schuppenförmige Teilchen umfassend:

   Metalloxidteilchen mit niedrigem Brechungsindex und Metalloxidmikroteilchen mit einem hohen Brechungsindex und einer mittleren Teilchengröße von 160-450 nm, die in den Teilchen mit niedrigem Brechungsindex in einer Menge von 5-50 Gewichts-% dispergiert sind,

   worin die schuppenförmigen Teilchen einen Lichtdiffusionsgrad von 80 oder mehr und eine totale Lichtdurchlässigkeit von unter 50% bei 550 nm haben,
   worin der hohe Brechungsindex der Mikroteilchen um 0,5 oder mehr höher ist als der niedrige Brechungsindex der Teilchen mit niedrigem Brechungsindex und worin der Lichtdiffusionsgrad definiert ist durch:

   Lichtdiffusionsgrad H = ((totale Lichtdurchlässigkeit Tt – direkte Lichtdurchlässigkeit Dt) / totale Lichtdurchlässigkeit Tt) x 100 = (Streulichtdurchlässigkeit St / totale Lichtdurchlässigkeit Tt) x 100.

2. Schuppenförmige Teilchen nach Anspruch 1, worin die schuppenförmigen Teilchen eine mittlere Teilchengröße von 5-500 $\mu$m, eine mittlere Dicke von 0,1-5 $\mu$m und ein mittleres Höhe-Breite-Verhältnis von 5-300 haben.

3. Schuppenförmige Teilchen nach Anspruch 1, worin die schuppenförmigen Teilchen eine mittlere Teilchengröße von 8-300 $\mu$m, eine mittlere Dicke von 0,2-2,5 $\mu$m und ein mittleres Höhe-Breite-Verhältnis von 8-200 haben.

4. Schuppenförmige Teilchen nach Anspruch 1, worin die schuppenförmigen Teilchen eine mittlere Teilchengröße von 8-50 $\mu$m, eine mittlere Dicke von 0,5-2,0 $\mu$m und ein mittleres Höhe-Breite-Verhältnis von 8-50 haben.

5. Schuppenförmige Teilchen nach einem beliebigen der Ansprüche 1 bis 4, worin die schuppenförmigen Teilchen eine totale Lichtreflexion von 40% oder mehr haben.

6. Schuppenförmige Teilchen nach einem beliebigen der Ansprüche 1 bis 5, worin die Hauptkomponente der Mikroteilchen zumindest eine ist, ausgewählt aus der Gruppe bestehend aus Zinkoxid (ZnO), Titandioxid (TiO$_2$), Zirkoniumoxid (ZrO$_2$), Ceroxid (CeO$_2$), Aluminiumoxid (Al$_2$O$_3$), Antimonoxid (Sb$_2$O$_3$) und Eisenoxid (Fe$_2$O$_3$).

7. Schuppenförmige Teilchen nach einem beliebigen der Ansprüche 1 bis 6, worin die Hauptkomponente der Teilchen mit niedrigem Brechungsindex die die Mikroteilchen enthalten, zumindest eine ist, ausgewählt aus der Gruppe bestehend aus Siliciumdioxid und Aluminiumsesquioxid.

8. Schuppenförmige Teilchen nach einem beliebigen der Ansprüche 1 bis 6, worin der hohe Brechungsindex der Mikroteilchen um 1,0 oder mehr höher ist als der niedrige Brechungsindex der Teilchen mit niedrigem Brechungsindex.

9. Schuppenförmige Teilchen nach einem beliebigen der Ansprüche 1 bis 8, worin die Kombination der Teilchen mit niedrigem Brechungsindex und der Mikroteilchen zumindest eine ist, ausgewählt aus der Gruppe bestehend aus den Kombinationen, ausgedrückt durch Teilchen von niedrigem Brechungsindex (Brechungsindex)/Mikroteilchen (Brechungsindex):

   Siliziumdioxid (Brechungsindex 1,46)/Titandioxid (Brechungsindex 2,72);
   Aluminiumoxid (Brechungsindex 1,76)/Eisensesquioxid (Brechungsindex 3,01);
   Siliziumdioxid (Brechungsindex 1,46)/Zinkoxid (Brechungsindex 2,1);
   Siliziumdioxid (Brechungsindex 1,46)/Zirkonoxid (Brechungsindex 2,1);
   Siliziumdioxid (Brechungsindex 1,46)/Ceroxid (Brechungsindex 2,2);
   Siliziumdioxid (Brechungsindex 1,46)/Eisensesquioxid (Brechungsindex 3,01).

**10.** Schuppenförmige Teilchen nach einem beliebigen der Ansprüche 1 bis 9, worin der Gehalt an Mikroteilchen in den schuppenförmigen Teilchen in einem Bereich von 8 bis 30 Gewichts-% liegt.

**11.** Schuppenförmige Teilchen nach einem beliebigen der Ansprüche 1 bis 10, worin die mittlere Teilchengröße der Mikroteilchen in einem Bereich von 200 bis 400 nm liegt.

**12.** Schuppenförmige Teilchen nach einem beliebigen der Ansprüche 1 bis 11, worin die schuppenförmigen Teilchen eine filmförmige gemahlene Mischung von geschmolzenem Glas und Metalloxidteilchen mit einem hohen Brechungsindex oder ein Sol-Gel-Produkt sind.

**13.** Kosmetikum enthaltend die schuppenförmigen Teilchen nach einem beliebigen der Ansprüche 1 bis 12.

**14.** Kosmetikum nach Anspruch 13, worin der Gehalt an schuppenförmigen Teilchen 1-70 Gewichts-% beträgt.

**15.** Beschichtungszusammensetzung umfassend schuppenförmige Teilchen nach einem beliebigen der Ansprüche 1 bis 12.

**16.** Beschichtungszusammensetzung nach Anspruch 15, worin der Gehalt an schuppenförmigen Teilchen 1-70 Gewichts-% beträgt.

**17.** Beschichtungsschicht umfassend eine gehärtete Applikation ("application") der Beschichtungszusammensetzung nach Anspruch 15 oder 16.

**18.** Harzzusammensetzung enthaltend schuppenförmige Teilchen nach einem beliebigen der Ansprüche 1 bis 12.

**19.** Harzzusammensetzung nach Anspruch 18, worin der Gehalt an schuppenförmigen Teilchen 1-70 Gewichts-% beträgt.

**20.** Harzform hergestellt durch Formen der Harzzusammensetzung nach Anspruch 18 oder 19.

**21.** Tintenzusammensetzung enthaltend die schuppenförmigen Teilchen nach einem beliebigen der Ansprüche 1 bis 12.

**22.** Tintenzusammensetzung nach Anspruch 21, worin der Gehalt an schuppenförmigen Teilchen 1-70 Gewichts-% beträgt.

**23.** Schuppenförmige Teilchen nach Anspruch 1, worin die besagte totale Lichtdurchlässigkeit oberhalb 30% liegt.

**Revendications**

**1.** Particules écailleuses comprenant :

des particules d'oxyde métallique possédant un indice de réfraction bas ; et
des microparticules d'oxyde métallique possédant un indice de réfraction élevé et une taille moyenne de particule de 160 à 450 nm dispersées à l'intérieur des particules possédant un indice de réfraction bas en une quantité de 5 à 50 % en poids,

dans lesquelles les particules écailleuses possèdent un degré de diffusion de lumière de 80 ou plus et une transmission de la lumière totale à 550 nm inférieure à 50 %,
dans lesquelles l'indice de réfraction élevé des microparticules est supérieur à l'indice de réfraction bas des particules possédant un indice de réfraction bas de 0,5 ou plus et
dans lesquelles le degré de diffusion de la lumière est défini par :

degré de diffusion de la lumière H = ((transmission de la lumière totale Tt – transmission de la lumière directe Dt) / transmission de la lumière totale Tt) x 100 = (transmission de la lumière diffusée St / transmission de la lumière totale Tt) x 100.

**2.** Particules écailleuses selon la revendication 1, dans lesquelles les particules écailleuses possèdent une taille moyenne de particule de 5 à 500 $\mu$m, une épaisseur moyenne de 0,1 à 5 $\mu$m, et un rapport hauteur/largeur moyen de 5 à 300.

**3.** Particules écailleuses selon la revendication 1, dans lesquelles les particules écailleuses possèdent une taille moyenne de particule de 8 à 300 $\mu$m, une épaisseur moyenne de 0,2 à 2,5 $\mu$m, et un rapport hauteur/largeur moyen de 8 à 200.

**4.** Particules écailleuses selon la revendication 1, dans lesquelles les particules écailleuses possèdent une taille moyenne de particule de 8 à 50 $\mu$m, une épaisseur moyenne de 0,5 à 2,0 $\mu$m, et un rapport hauteur/largeur moyen de 8 à 50.

**5.** Particules écailleuses selon l'une quelconque des revendications 1 à 4, dans lesquelles les particules écailleuses possèdent un pouvoir réflecteur de la lumière total de 40 % ou plus.

**6.** Particules écailleuses selon l'une quelconque des revendications 1 à 5, dans lesquelles le composant principal des microparticules est au moins un composant choisi dans le groupe constitué par l'oxyde de zinc (ZnO), le dioxyde de titane ($TiO_2$), l'oxyde de zirconium ($ZrO_2$), l'oxyde de cérium ($CeO_2$), l'oxyde d'aluminium ($Al_2O_3$), l'oxyde d'antimoine ($Sb_2O_3$), et l'oxyde de fer ($Fe_2O_3$).

**7.** Particules écailleuses selon l'une quelconque des revendications 1 à 6, dans lesquelles le composant principal des particules possédant un indice de réfraction bas contenant les microparticules est au moins un composant choisi dans le groupe constitué par le dioxyde de silicium et le sesquioxyde d'aluminium.

**8.** Particules écailleuses selon l'une quelconque des revendications 1 à 6, dans lesquelles l'indice de réfraction élevé des microparticules est supérieur à l'indice de réfraction bas des particules possédant un indice de réfraction bas de 1,0 ou plus.

**9.** Particules écailleuses selon l'une quelconque des revendications 1 à 8, dans lesquelles la combinaison des particules possédant un indice de réfraction bas et des microparticules est au moins une combinaison choisie dans le groupe constitué par les combinaisons, indiquées sous la forme des particules possédant un indice de réfraction bas (indice de réfraction)/micro-particules (indice de réfraction) :

dioxyde de silicium (1,46 d'indice de réfraction)/dioxyde de titane (2,72 d'indice de réfraction) ;
oxyde d'aluminium (1,76 d'indice de réfraction)/sesquioxyde de fer (3,01 d'indice de réfraction) ;
dioxyde de silicium (1,46 d'indice de réfraction)/oxyde de zinc (2,1 d'indice de réfraction) ;
dioxyde de silicium (1,46 d'indice de réfraction)/oxyde de zirconium (2,1 d'indice de réfraction) ;
dioxyde de silicium (1,46 d'indice de réfraction)/oxyde de cérium (2,2 d'indice de réfraction) ;
dioxyde de silicium (1,46 d'indice de réfraction) /sesquioxyde de fer (3,01 d'indice de réfraction).

**10.** Particules écailleuses selon l'une quelconque des revendications 1 à 9, dans lesquelles la teneur en microparticules dans les particules écailleuses se situe dans la plage de 8 à 30% en poids.

**11.** Particules écailleuses selon l'une quelconque des revendications 1 à 10, dans lesquelles la taille moyenne de particule des microparticules se situe dans la plage de 200 à 400 nm.

**12.** Particules écailleuses selon l'une quelconque des revendications 1 à 11, dans lesquelles les particules écailleuses sont un mélange broyé de particules d'oxyde métallique et de verre fondu mis en forme de film possédant un indice de réfraction élevé, ou un produit sol-gel.

**13.** Produit cosmétique contenant des particules écailleuses selon l'une quelconque des revendications 1 à 12.

**14.** Produit cosmétique selon la revendication 13, dans lequel la teneur en particules écailleuses est de 1 à 70 % en poids.

**15.** Composition de revêtement comprenant des particules écailleuses selon l'une quelconque des revendications 1 à 12.

**16.** Composition de revêtement selon la revendication 15, dans laquelle la teneur en particules écailleuses est de 1 à 70 % en poids.

**17.** Couche de revêtement comprenant une application durcie de la composition de revêtement selon la revendication 15 ou 16.

**18.** Composition de résine contenant des particules écailleuses selon l'une quelconque des revendications 1 à 12.

**19.** Composition de résine selon la revendication 18, dans laquelle la teneur en particules écailleuses est de 1 à 70 % en poids.

**20.** Forme de résine produite en moulant la composition de résine selon la revendication 18 ou 19.

**21.** Composition d'encre contenant des particules écailleuses selon l'une quelconque des revendications 1 à 12.

**22.** Composition d'encre selon la revendication 21, dans laquelle la teneur en particules écailleuses est de 1 à 70 % en poids.

**23.** Particules écailleuses selon la revendication 1, dans laquelle ladite transmission de lumière totale est supérieure à 30 %.

# Fig. 1

EP 1 512 729 B1